# EUROPÄISCHE PATENTSCHRIFT

(11) **EP 0 450 123 B1**
(45) Veröffentlichungstag und Bekanntmachung des Hinweises auf die Patenterteilung: **05.07.1995**
(21) Anmeldenummer: 90106549.0
(22) Anmeldetag: 05.04.1990
(51) Int. Cl.: A61K 31/195, A61K 47/38

(54) **Diclofenac-Natrium enthaltende pharmazeutische Zusammensetzung zur topischen Anwendung**
Pharmaceutical composition for topical administration containing diclofenac-sodium
Composition pharmaceutique pour application topique contenant du diclofenac-sodium

(43) Veröffentlichungstag der Anmeldung: 09.10.1991
(73) Patentinhaber: Knoll AG, 67061 Ludwigshafen (DE)
(72) Erfinder:
(74) Vertreter: Goldscheid, Bettina

(56) Entgegenhaltungen:
- EP-A- 0 185 374
- EP-A- 0 206 291
- DE-A- 2 504 615
- DE-A- 3 400 106
- LU-A- 85 145
- INTERNATIONAL JOURNAL OF PHARMACEUTICS, Band 24, Nr 1, Januar 1985, Seiten 115-124, Elsevier Science Publishers B.V. (Biomedical Division), Amsterdam, NL; S.-I. NAITO et al.: "Percutaneous absorption of diclofenac sodium ointment"
- PATENT ABSTRACTS OF JAPAN, Band 13, Nr. 15 (C-559)[3363], 13. Januar 1989;& JP-A-63 222 121 (NIPPON SODA CO., LTD) 16-09-1988

## Beschreibung

Die Erfindung betrifft eine Diclofenac-Natrium enthaltende pharmazeutische Zusammensetzung zur topischen Anwendung bei Erkrankungen des rheumatischen Formenkreises.

Diclofenac (2-(2,6-Dichloranilino)-phenylessigsäure) ist als nicht stereoides Antirheumatikum, Antiphlogistikum und Schmerzmittel seit vielen Jahren bekannt (vgl. DE-PS 1793592); trotz seiner hohen Wirksamkeit und lange andauernden Wirkung ist seine orale Anwendung aber infolge gravierender Nebenwirkungen, wie z.B. Magen-Darmblutungen, Herz- und Nierenfunktionsschädigungen stark eingeschränkt.

Zur Vermeidung oder Verringerung von Nebenwirkungen ist es deshalb wünschwenswert, die orale Applikation durch eine topische Applikation zu ersetzen, insbesondere bei örtlich lokalisierten Erkrankungen oder Verletzungen des Bewegungsapparates.

Für eine topische Anwendung, d.h. Abgabe an und durch die Haut über einen längeren Zeitraum, wurden medizinische Pflaster vorgeschlagen, die als Wirkstoff ein Antiphlogistikum, z.B. Diclofenac, enthalten (vgl. DE-A-3344691, 3347277, 3347278, 3409079, EP-A-0283434).

Aus der DE-A-3720896 sind pharmazeutische Zubereitungen zur topischen Anwendung bekannt, die Diclofenac oder ein pharmazeutisch verwendbares Salz davon zusammen mit einer die Permeationsfähigkeit des Wirkstoffs verstärkenden Verbindung enthalten. Aus der DE-A-2935776 sind organische Salze des Diclofenacs, z.B. das Diethylaminsalz, bekannt die z.B. in einer Salbengrundlage, Emulsion, in einem Lösungsmittel oder Gel verarbeitet als Arzneimittel vorliegen.

Aus der EP-A-0206291 sind aufsprühbare pharmazeutische Zubereitungen für die topische Anwendung bekannt, die Diclofenac in einem Lösungsmittelgemisch aus einem oder mehreren flüchtigen, physiologisch verträglichen Lösungsmitteln und einem oder mehreren nicht flüchtigen, physiologisch verträglichen Lösungsmitteln, z.B. mehrwertigen Alkoholen, wie Propylenglycol, Glycerin, oder flüssige Polyethylenglycole, enthalten; vorzugsweise enthalten diese Zusammensetzungen auch eine filmbildende Substanz, z.B. Polyacrylat.

Die EP-A-0185374 beschreibt flüssige Diclofenac-Zubereitungen, die aus einer Lösung von Diclofenac oder einem seiner Salze in einer Mischung aus Propylenglycol, Polyethylenglycol und Wasser bestehen.

Aus der EP-A-0147476 sind Gelzusammensetzungen zur externen Applikation bekannt, die Diclofenac-Natrium als Wirkstoff, Wasser, niedrige Alkanole und Glycole als Lösungsmittel, und ein Carboxyvinylpolymeres als Gelbildner, sowie eine schwache basische Substanz, wie ein aliphatisches Amin, enthalten.

Die DE-A-3336047 beschreibt topisch applizierbare pharmazeutische Zusammensetzungen auf der Basis einer O/W-Emulsion, die als Wirkstoff eine nicht-stereoide, antiinflammatorisch wirksame Verbindung mit mindestens einer Säuregruppe, z.B. Diclofenac, enthalten, und die die Eigenschaften eines Gels mit denen einer O/W-Emulsion vereinigen; die Zusammensetzung enthält 3 bis 15 Gew. % eines Lipids (Fettphase), und falls die Lipidphase nicht selbst emulgierend ist, 0.5 bis 5 Gew. % eines Emulgators, sowie 0.5 bis 2 Gew. % eines Gelgerüstbildners. Als Gelgerüstbildner wird vorzugsweise ein Polyacrylat verwendet, und als Base zur Neutralisation der sauren Gruppen ein organisches Amin, z.B. Diethylamin.

Ein Nachteil der pharmazeutischen Zusammensetzungen gemäß der vorstehend genannten DE-A-3336047, die sich als topisch anwendbare Präparate auf dem Markt befinden, ist die Gegenwart von Diethylamin, das insbesondere auch bei der Verwendung von Polyacrylaten als Gelgerüstbildner zur Neutralisation zugesetzt werden muß. Als sekundäres aliphatisches Amin gehört Diethylamin zu den chemischen Verbindungen , die bei Kontakt mit nitrosierenden Agentien, wie z.B. Nitrit oder Stickoxiden der Luft während der Herstellung, Lagerung, Verarbeitung und auch im Organismus N-Nitrosamine bilden können; auch in extern anzuwendenden Arzneimitteln können solche sekundäre aliphatische Amine mit Stickoxiden der Luft chemisch reagieren und N-Nitrosamine bilden, die die menschliche Haut durchdringen können. Die Elimination von sekundären aliphatischen Aminen, wie z.B. von Diethylamin, aus Arzneimitteln stellt deshalb ein dringendes Bedürfnis dar, wobei diese Forderung insbesondere auch für extern anwendbare Antirheumatika/Antiphlogistika gilt, weil solche Mittel in der Regel langfristig angewandt werden müssen, und ihre Galenik auf eine gute Penetration ausgerichtet ist.

In dem "International Journal of Pharmaceutics", Bd. 24, Nr. 1, Januar 1985, Seiten 115-124,sind topische Diclofenac-Zubereitungen angegeben, die u.a. auf Basis eines niedrigen Anteils von Propylenglykol, Isopropylmyristat und Cellulosederivaten formuliert sind.

Aufgabe der vorliegenden Erfindung ist es eine Diclofenac enthaltende pharmazeutische Zusammensetzung zur topischen Anwendung bereitzustellen, die die vorstehend genannten Nachteile (insbesondere die Möglichkeit der N-Nitrosamin-Bildung aufgrund der Gegenwart von Aminen) nicht aufweist, die eine gute Bioverfügbarkeit (Resorption) nach externer Anwendung und eine therapeutisch wirksame Konzentration am Zielort (Gelenk, Muskel- oder Weichteilschwellung) gewährleistet, eine gute Stabilität über einen längeren Zeitraum besitzt, und die sich leicht und in reproduzierbarer Weise zur äußerlichen Anwendung auf der Haut eignet.

Es wurde nun gefunden, daß diese Aufgabenstellung erfindungsgemäß mit einer Diclofenac-Natrium enthaltenden pharmazeutischen Zusammensetzung in Form eines Gels gelöst werden kann.

Gegenstand der Erfindung ist eine Diclofenac-Natrium enthaltende pharmazeutische Zusammensetzung nach Anspruch 1 zur topischen Anwendung in Form eines Gels, die dadurch gekennzeichnet ist, daß sie als Gelbildner Hydroxypropylcellulose und/oder Hydroxypropylmethylcellulose in einer Menge von 1 bis 20 Gew.% sowie Propylenglykol in einer Menge von 15 bis 35 Gew.% enthält, wobei die Zusammensetzung von Isopropylmyristat frei ist, ggf.zusammen mit Mitteln zur pH-Wert-Einstellung, Stabilisierung, Konservierung und/oder üblichen pharmazeutischen Hilfs- und/oder Trägerstoffen.
Bei der erfindungsgemäßen pharmazeutischen Zusammensetzung handelt es sich um ein reines, transparenten Gel; es enthält keine emulgierte Fettphase, und benötigt deshalb auch keinen Emulgator. Es hat sich außerdem gezeigt, daß unter Verwendung eines erfindungsgemäß eingesetzten Cellulosederivats als Gelbildner ein Gel erhalten wird, in dem das Diclofenac als Natriumsalz besser löslich ist, als z.B. in einem Gel auf der Basis eines anderen Gelbildners, wie z.B. eines Polyacrylats; aufgrund dieser Eigenschaft läßt sich das Diclofenac in dem erfindungsgemäßen Gel leicht einarbeiten, und es ist deshalb nicht notwendig, ein Salz eines organischen Amins, wie z.B. das Diclofenac-Diethylammoniumsalz, zu verwenden.

Bevorzugte Ausgestaltungen des erfindungsgemäßen Gels sind Gegenstand der Ansprüche 2 bis 14.

Das als Gelbildner erfindungsgemäß verwendete Cellulosederivat stellt Hydroxypropyl-, Hydroxypropylmethylcellulose dar. Das Gel enthält den Gelbildner in einer Menge von 1 bis 20, insbesondere von 1 bis 10 und in erster Linie von 2 bis 6 Gew.%, bezogen auf die Gesamtzusammensetzung (Gel); die zweckmäßigste Menge hängt dabei insbesondere von der Viskosität des Gelbildners ab.

Die erfindungsgemäßen pharmazeutischen Zusammensetzungen in Form eines Gels können für derartige Zusammensetzungen an sich übliche weitere pharmazeutische Hilfs- und/oder Trägerstoffe enthalten; vorzugsweise enthalten sie Stabilisatoren, Antioxidantien, Lösungsvermittler, Rückfettungsmittel, Konservierungsmittel, und/oder Parfüms; zur Förderung des Penetrationsvermögens ist es auch insbesondere bevorzugt, einen Penetrationsförderer zuzusetzen, wie z.B. Propylenglycol und/oder andere penetrationsfördernde, für derartige Applikationen üblicherweise verwendete Mittel, und vorzugsweise in den dafür üblichen Mengen.

Das erfindungsgemäße Gel enthält als organische Lösungsmittelkomponente Propylenglykol sowie gegebenenfalls Ethanol, Isopropylalkohol und/oder Polyethylenglycol 400, wobei insbesondere eine Kombination von 2 oder mehreren dieser Lösungsmittel bevorzugt ist. Isopropylalkohol und/oder Ethanol werden vorzugsweise in einer Menge von 1 bis 35, insbesondere 3 bis 20 und in erster Linie 3 bis 10 Gew. %, bezogen auf die Gesamtzusammensetzung (Gel) verwendet; Propylenglycol liegt in einer Menge von 15 bis 35 Gew.%; und
Polyethylenglycol 400 vorzugsweise in einer Menge von 1 bis 10, insbesondere 2 bis 5 Gew. %, vor. Durch das Vorhandensein von Ethanol, Propylenglycol und/oder Isopropylalkohol läßt sich ein kühlender Effekt beim Aufbringen des Gels auf die Haut erzielen, außerdem zeigen insbesondere Propylenglycol und Isopropylalkohol auch eine penetrationsfördernde Wirkung und besitzen auch autokonservierende Eigenschaften für das Gel.

Das erfindungsgemäße Gel enthält vorzugsweise auch Substanzen zur Förderung der Hautverträglichkeit, insbesondere PEG-7-Glyceryl-cocoat (Cetiol^{R} HE, von der Firma Henkel, Düsseldorf), Stabilisatoren, insbesondere Natriumdisulfit, und Substanzen zur pH-Einstellung des Gels, insbesondere Natriumhydroxid. Das PEG-7-Glyceryl-cocoat liegt vorzugsweise in einer Menge von 0.5 bis 10, insbesondere 1 bis 5, und in erster Linie 2 bis 4 Gew. vor; das Natriumdisulfit vorzugsweise in einer Menge von 0.01 bis 0.2, insbesondere 0.05 bis 0.1 Gew. %, bezogen auf die Gesamtzusammensetzung (Gel). Zweckmäßigerweise enthält das erfindungsgemäße Gel eine Kombination von PEG-7-Glyceryl-cocoat und Natriumdisulfit. Das Mittel zur pH-Wert-Einstellung wird in der zur Einstellung des gewünschten pH-Wertes, der im allgemeinen im Bereich von 7.0 bis 8.0 liegt, erforderlichen Menge verwendet.

Das erfindungsgemäße Gel enthält das Diclofenac-Natrium vorzugsweise in einer Menge von 0.1 bis 10 Gew. %, insbesondere in einer Menge von 0.2 bis 5 Gew. %, und in erster Linie von 0.5 bis 2 Gew. %. Gegebenenfalls können die Gele neben dem eigentlichen Wirkstoff Diclofenac-Natrium auch noch weitere pharmazeutische Wirkstoffe, die mit Diclofenac-Natrium im Rahmen der Applikation verträglich sind, enthalten, z.B. Wirkstoffe zur Therapie von Hauterkrankungen (Dermatosen), Antibiotika, Sulfonamide, Desinfektionsmittel, Corticoide, Hautpflegemittel, und/oder durchblutungsfördernde (hyperämisierende) Mittel, wie z.B. Nicotinsäureester (z.B. Benzylnicotinat), Salicylsäureester (z.B. Bornylsalicylat), Extractum Capsici fructus, und/oder etherische Öle (z.B. Fichtennadelöl).

Ein aufgrund seiner Eigenschaften und seiner antirheumatischen/antiphlogistischen Wirkung besonders bevorzugtes erfindungsgemäßes Gel enthält die folgende Bestandteile: Diclofenac-Natrium/Hydroxypropylmethylcellulose, Hydroxyethylcellulose und/oder Hydroxypropylcellulose/Propylenglycol, Isopropylalkohol, Ethanol und/oder Polyethylenglycol 400/PEG-7-Glyceryl-cocoat/Natriumdisufit/Natriumhydroxid/Wasser, insbesondere in den für die einzelnen Komponenten vorstehend genannten bevorzugten Mengenverhältnissen.

Die Herstellung der erfindungsgemäßen Zusammensetzungen (Gele) kann auf eine für die Herstellung derartiger Gele ansich übliche, allgemein bekannte Weise erfolgen.

Gegenstand der Erfindung ist auch ein Verfahren zur Herstellung der erfindungsgemäßen Zusammensetzungen (Gele) gemäß Anspruch 12, das dadurch gekennzeichnet ist, daß man den Wirkstoff, den Gelbildner und die übrigen Bestandteile in Wasser löst; zweckmäßigerweise arbeitet man dabei unter Erwärmen und nachfolgender Abkühlung. Zum Schutz vor Oxidation arbeitet man vorzugsweise unter einer Inertgasatmosphäre, insbesondere unter Stickstoff.

In den nachfolgenden Beispielen wird eine erfindungsgemäße Zusammensetzung (Gel) und das Verfahren zu ihrer Herstellung beschrieben, ohne die Erfindung auf diese Ausführungsformen zu beschränken. Vorstehend und nachfolgend beziehen sich Prozent- und Mengenangaben, wenn nicht anders angegeben, auf das Gewicht.

### BEISPIELE

### Beispiel 1

### Herstellung eines Diclofenac-Gels

Ansatz: 100 kg

| Bestandteil | Menge in kg |
|---|---|
| Diclofenac-Natrium | 1.00 |
| Hydroxypropylmethylcellulose (Qualität entsprechend USP XXI, Methocel E4M Premium, von der Firma Colorcon) | 2.30 |
| Propylenglycol (DAB 9) | 25.00 |
| PEG-7-Glyceryl-cocoat (Cetiol^{R} HE, von der Firma Henkel, Düsseldorf) | 3.00 |
| Isopropylalkohol (entsprechend DAC 86, 2. Lief. 87) | 5.00 |
| Natriumdisulfit (entsprechend USP XXI, NF 16) | 0.08 |
| Natriumhydroxid (DAB 9) | 0.03 |
| Wasser, gereinigt (DAB 9) | 63.59 |

Die Bestandteile werden unter Stickstoffatmosphäre (USP XXI/NF 16) unter Rühren und kurzem Erwärmen gelöst; nach dem Abkühlen wird ein farbloses, klares Gel erhalten, das schwach nach Isopropylalkohol riecht. Der pH-Wert (direkt gemessen) beträgt 7.4 bis 7.9.

Das Gel wird in Aluminiumtuben (50 g und 100 g-Packungen) mit Epoxid-Innenlackierung abgefüllt.

### Beispiel 2

Nach dem im Beispiel 1 angegebenen Verfahren wird ein Diclofenac-Gel mit den nachstehend angegebenen Bestandeilen hergestellt.
Ansatz: 100 g

| Bestandteil | Menge in g |
|---|---|
| Diclofenac-Natrium | 1.0 |
| Hydroxypropylcellulose | 4.0 |
| Propylenglycol | 20.0 |
| Isopropanol | 10.0 |
| Polyethylenglycol 400 | 3.0 |
| PEG-7-Glyceryl-cocoat | 3.0 |
| Natriumdisulfit | 0.1 |
| Natriumhydroxid | 0.04 |
| Ger. Wasser | ad 100.00 |

Nach Anwendung des im vorstehenden Beispiel 1 hergestellten erfindungsgemäßen "Diclofenac-Gels" (Wirkstoffgehalt 1 Gew. %) und eines im Handel befindlichen Vergleichspräparats (gemäß der DE-A-3336047, mit 1.16 Gew. % Diclofenac-Diethylammonium als Wirkstoff und Polyacrylat als Gelbildner) auf der Rückenhaut von Propanden wurde die Pharmakokinetik der Blutspiegel für beide Präparate bestimmt. Überraschenderweise zeigte sich, daß mit dem erfindungsgemäßen Gel eine um ca. 70 % höhere Bioverfügbarkeit erreicht wird als mit dem im Handel befindlichen Gel.

In einer klinischen Prüfung wurde nachgewiesen, daß der Wirkstoff Diclofenac-Natrium nach Anwendung des erfindungsgemäßen Gels auf den Kniegelenk von Patienten durch die Haut in die Synovialflüssigkeit penetriert. Die Konzentration von Diclofenac-Natrium in der Synovialflüssigkeit wurde gemessen.

Die Wirksamkeit des erfindungsgemäßen Gels wurde in einer randomisierten placebokontrollierten Doppelblindstudie nachgewiesen. Hierbei wurden insgesamt 30 Patienten mit frischem stumpfen Trauma des Sprunggelenks mit dem erfindungsgemäßen Gel (bzw. der Gelgrundlage als Placebo) 14 Tage lang behandelt. Die Prüfpräparate wurden 30 mal täglich über der Verletzungsstelle aufgetragen, wobei jedesmal ca. 3g Gel (30 mg Diclofenac-Natrium) leicht in die Haut des verletzten Fußes eingerieben wurde. Wegen der Empfindlichkeit der Beinvolumenmessung, die zur Verlaufkontrolle des Schwellungszustandes diente, durften keine elastischen Binden angelegt werden.

Als Hauptzielkriterien dienten der Schwellungsrückgang in der verletzten Region, ein Schmerzscore, der sich aus Ruhe- und Bewegungsschmerz errechnete, sowie die globale Beurteilung des Therapieerfolges. Nebenzielkriterien waren Kreislaufparameter, die Verträglichkeit und Handhabbarkeit des Gels. Die Bestimmung des Schwellungsvolumens erfolgte mit einer hochempfindlich Messapparatur über die Messung des verdrängten Flüssigkeitsvolumens (Wasser). Zur Beschreibung der Schwellung wird dabei die Volumendifferenz zwischen krankem und gesundem Bein herangezogen und als "Schwellungsvolumen" bezeichnet.

Die biometrische Auswertung der Ergebnisse ergab eine signifikante Überlegenheit der Behandlung mit Verum (erfindungsgemäßes Gel) gegenüber Placebo in den folgenden Bereichen:
Nach Verabreichung des erfindungsgemäßen Gels klang die Schwellung bereits nach zwei Tagen ab, während unter Placebo noch eine Zunahme zu verzeichnen war; beim Schmerzscore betrug der Unterschied zwischen Verum und Placebo am zweiten Tag einen Schweregrad, und dieser Unterschied bestnd auch noch nach einer Woche; der Therapieerfolg wurde bereits am zweiten Tag mit dem erfindungsgemäßen Gel besser beurteilt, und diese günstigere Beurteilung hielt über den ganzen Behandlungszeitraum an.

Die subjektive Verträglichkeit war insgesamt gut; die Einreibbarkeit des Gels wurde als "leicht" bis mäßig" beurteilt.

Wesentlich für die Beurteilung der Verträglichkeit des erfindungsgemäßen Gels im Vergleich zu dem Vergleichspräparat ist die Abwesenheit von Diethylamin im erfindungsgemäßen Gel, wodurch die Möglichkeit der unbedingt zu vermeidenden N-Nitrosaminbildung ausgeschaltet werden kann. Durch das Fehlen von Emulgatoren im erfindungsgemäßen Gel (gegnüber dem Vergleichspräparat) kann auch die mit Emulgatoren oft verbundene Austrocknung der Haut vermieden werden.

Mit dem erfindungsgemäßen Gel wird somit eine Diclofenac enthaltende pharmazeutische Zusammensetzung zur topischen Anwendung bereitgestellt, die frei ist von Aminen (Ausschluß der Möglichkeit einer N-Nitrosaminbildung), die eine gute Bioverfügbarkeit (Resorption) nach externer Anwendung zeigt, eine gute Stabilität über einen längeren Zeitraum besitzt, und sich leicht und in reproduzierbarer Weise zur äußerlichen Anwendung auf der Haut eignet.

Gegenstand der vorliegende Erfindung ist es deshalb auch eine erfindungsgemäße pharmazeutische Zusammensetzung (Gel) zur Verwendung als antirheumatisch, antiphlogistisch und/oder antiinflammatorisch wirkendes Arzneimittel bei der Behandlung entsprechender Erkrankungen.

Die Art, Dosis und Häufigkeit der topischen Verabreichung richtet sich insbesondere nach der Schwere der Erkrankung und dem Allgemeinzustand des Patienten, aber auch nach dem Zustand und der Empfindlichkeit der Haut. In der Regel entspricht die Verabreichung den für derartige Zusammensetzungen üblichen Bedingungen.

## Patentansprüche (Patentansprüche für folgende(n) Vertragsstaat(en): AT, BE, CH, DE, DK, FR, GB, IT, LI, LU, NL, SE)

1. Diclofenac-Natrium in einer Menge von 0,1 bis 10 Gew.% enthaltende pharmazeutische Zusammensetzung zur topischen Anwendung in Form eines Gels, die als Gelbildner Hydroxypropylcellulose und/oder Hydroxypropylmethylcellulose in einer Menge von 1 bis 20 Gew.% sowie Propylenglykol in einer Menge von 15 bis 35 Gew.% enthält, wobei die Zusammensetzung von Isopropylmyristat frei ist, gegebenenfalls zusammen mit Mitteln zur pH-Wert-Einstellung, Stabilisierung, Konservierung und/oder üblichen pharmazeutischen Hilfs- und/oder Trägerstoffen.

2. Zusammensetzung nach Anspruch 1, dadurch **gekennzeichnet,** dass sie Stabilisatoren, Antioxidantien, Lösungsvermittler, Rückfettungsmittel, Konservierungsmittel und/oder Parfüms enthält.

3. Zusammensetzung nach Anspruch 2, dadurch **gekennzeichnet,** dass sie PEG-7-Glyceryl-cocoat, Isopropylalkohol und/oder Natriumdisulfit enthält.

4. Zusammensetzung nach Anspruch 3, dadurch **gekennzeichnet,** dass sie das Diclofenac-Natrium in einer Menge von 0,2 bis 5 Gew.%, insbesondere in einer Menge von 0,5 bis 2 Gew.%, enthält.

5. Zusammensetzung nach einem der Ansprüche 1 bis 4, dadurch **gekennzeichnet,** dass sie Diclofenac-Natrium, Hydroxypropylmethylcellulose, Propylenglykol, PEG-7-Glyceryl-cocoat, Isopropylalkohol und Natriumdisulfit enthält.

6. Zusammensetzung nach einem der Ansprüche 1 bis 5, dadurch **gekennzeichnet,** dass sie ein penetrationsförderndes Mittel enthält.

7. Zusammensetzung nach einem der Ansprüche 1 bis 6, dadurch **gekennzeichnet,** dass sie als organische Lösungsmittelkomponenten Ethanol, Isopropylalkohol und/oder Polyethylenglykol 400 enthält.

8. Zusammensetzung nach einem der Ansprüche 1 bis 7, dadurch **gekennzeichnet,** dass sie Isopropylalkohol und/oder Ethanol in einer Menge von 1 bis 35 Gew.%, vorzugsweise 3 bis 20 Gew.%, und insbesondere 3 bis 10 Gew.%, bezogen auf die gesamte Zusammensetzung, enthält.

9. Zusammensetzung nach einem der Ansprüche 1 bis 8, dadurch **gekennzeichnet,** dass sie Polyethylenglykol 400 in einer Menge von 1 bis 10, vorzugsweise 2 bis 5 Gew.%, bezogen auf die gesamte Zusammensetzung, enthält.

10. Zusammensetzung nach einem der Ansprüche 1 bis 9, dadurch **gekennzeichnet,** dass sie PEG-7-Glyceryl-cocoat in einer Menge von 0,5 bis 10, vorzugsweise 1 bis 5 und insbesondere 2 bis 4 Gew.%, bezogen auf die gesamte Zusammensetzung, enthält.

11. Zusammensetzung nach einem der Ansprüche 1 bis 10, dadurch **gekennzeichnet,** dass sie Natriumdisulfit in einer Menge von 0,01 bis 0,2, vorzugsweise 0,05 bis 0,1 Gew.%, bezogen auf die gesamte Zusammensetzung, enthält.

12. Verfahren zur Herstellung einer Zusammensetzung nach einem der Ansprüche 1 bis 11, dadurch **gekennzeichnet,** dass man den Wirkstoff, den Gelbildner und die übrigen Bestandteile in Wasser löst, gegebenenfalls unter Erwärmen und nachfolgendem Abkühlen.

13. Verfahren nach Anspruch 12, dadurch **gekennzeichnet,** dass man die Lösung unter einer Inertgasatmosphäre, insbesondere unter Stickstoff, durchführt.

14. Pharmazeutische Zusammensetzung nach einem der Ansprüche 1 bis 13 zur Verwendung als antirheumatisch, antiphlogistisch und/oder antiinflammatorisch wirkendes Arzneimittel.

## Patentansprüche (Patentansprüche für folgende(n) Vertragsstaat(en): ES, GR)

1. Verfahren zur Herstellung einer Diclofenac-Natrium in einer Menge von 0,1 bis 10 Gew.% enthaltenden pharmazeutischen Zusammensetzung zur topischen Anwendung in Form eines Gels, die als Gelbildner Hydroxypropylcellulose und/oder Hydroxypropylmethylcellulose in einer Menge von 1 bis 20 Gew.% sowie Propylenglykol in einer Menge von 15 bis 35 Gew.% enthält, wobei die Zusammensetzung von Isopropylmyristat frei ist, gegebenenfalls zusammen mit Mitteln zur pH-Wert-Einstellung, Stabilisierung, Konservierung und/oder üblichen pharmazeutischen Hilfs- und/oder Trägerstoffen, indem man den Wirkstoff, den Gelbildner und die übrigen Bestandteile in Wasser löst, gegebenenfalls unter Erwärmen und nachfolgendem Abkühlen.

2. Verfahren nach Anspruch 1, dadurch ***gekennzeichnet*****,** daß die Zusammensetzung Stabilisatoren, Antioxidantien, Lösungsvermittler, Rückfettungsmittel, Konservierungsmittel und/oder Parfüms enthält.

3. Verfahren nach Anspruch 2, dadurch ***gekennzeichnet*****,** daß die Zusammensetzung PEG-7-Glyceryl-cocoat, Isopropylalkohol und/oder Natriumdisulfit enthält.

4. Verfahren nach Anspruch 3, dadurch ***gekennzeichnet*****,** daß die Zusammensetzung das Diclofenac-Natrium in einer Menge von 0,2 bis 5 Gew.%, insbesondere in einer Menge von 0,5 bis 2 Gew.%, enthält.

5. Verfahren nach einem der Ansprüche 1 bis 4, dadurch ***gekennzeichnet*****,** daß die Zusammensetzung Diclofenac-Natrium, Hydroxypropylmethylcellulose, Propylenglykol, PEG-7-Glayceryl-cocoat, Isopropylalkohol und Natriumdisulfit enthält.

6. Verfahren nach einem der Ansprüche 1 bis 5, dadurch ***gekennzeichnet*****,** daß die Zusammensetzung ein penetrationsförderndes Mittel enthält.

7. Verfahren nach einem der Ansprüche 1 bis 6, dadurch ***gekennzeichnet*****,** daß die Zusammensetzung als organische Lösungsmittelkomponenten Ethanol, Isopropylalkohol und/oder Polyethylenglykol 400 enthält.

8. Verfahren nach einem der Ansprüche 1 bis 7, dadurch ***gekennzeichnet*****,** daß die Zusammensetzung Isopropylakkohol und/oder Ethanol in einer Menge von 1 bis 35 Gew.%, vorzugsweise 3 bis 20 Gew.%, und insbesondere 3 bis 10 Gew.%, bezogen auf die gesamte Zusammensetzung, enthält.

9. Verfahren nach einem der Ansprüche 1 bis 8, dadurch ***gekennzeichnet*****,** daß die Zusammensetzung Polyethylenglykol 400 in einer Menge von 1 bis 10, vorzugsweise 2 bis 5 Gew.%, bezogen auf die gesamte Zusammensetzung, enthält.

10. Verfahren nach einem der Ansprüche 1 bis 9, dadurch ***gekennzeichnet*****,** daß die Zusammensetzung PEG-7-Glyceryl-cocoat in einer Menge von 0,5 bis 10, vorgusweise 1 bis 5 und insbesondere 2 bis 4 Gew.%, bezogen auf die gesamte Zusammensetzung, enthält.

11. Verfahren nach einem der Ansprüche 1 bis 10, dadurch ***gekennzeichnet*****,** daß die Zusammensetzung Natriumdisulfit in einer Menge von 0,01 bis 0,2, vorzugsweise 0,05 bis 0,1 Gew.%, bezogen auf die gesamte Zusammensetzung, enthält.

## Claims (Claims for the following Contracting State(s): AT, BE, CH, DE, DK, FR, GB, IT, LI, LU, NL, SE)

1. Pharmaceutical composition containing diclofenac sodium in an amount from 0.1 to 10 wt.% for topical application in the form of a gel, which contains as gel former, hydroxypropylcellulose and/or hydroxypropylmethylcellulose in an amount from 1 to 20 wt.%, and propylene glycol in an amount from 15 to 35 wt.%, the composition being free of isopropyl myristate, optionally together with means for adjusting the pH value, stabilisation, preservation and/or conventional pharmaceutical auxiliaries and/or excipients.

2. Composition according to claim 1, characterised in that it contains stabilisers, anti-oxidants, solubilisers, reverse-fatting agents, preservatives and/or perfumes.

3. Composition according to claim 2, characterised in that it contains PEG-7-glyceryl cocoate, isopropyl alcohol and/or sodium disulphite.

4. Composition according to claim 3, characterised in that it contains the diclofenac sodium in an amount from 0.2 to 5 wt.%, in particular in an amount from 0.5 to 2 wt.%.

5. Composition according to one of claims 1 to 4, characterised in that it contains diclofenac sodium, hydroxypropylmethylcellulose, propylene glycol, PEG-7-glyceryl cocoate, isopropyl alcohol and sodium disulphite.

6. Composition according to one of claims 1 to 5, characterised in that it contains a penetration-promoting agent.

7. Composition according to one of claims 1 to 6, characterised in that it contains ethanol, isopropyl alcohol and/or polyethylene glycol 400 as organic solvent components.

8. Composition according to one of claims 1 to 7, characterised in that it contains isopropyl alcohol and/or ethanol in an amount from 1 to 35 wt.%, preferably 3 to 20 wt.%, and in particular 3 to 10 wt.%, based on the total composition.

9. Composition according to one of claims 1 to 8, characterised in that it contains polyethylene glycol 400 in an amount from 1 to 10, preferably 2 to 5 wt.%, based on the total composition.

10. Composition according to one of claims 1 to 9, characterised in that it contains PEG-7-glyceryl cocoate in an amount from 0.5 to 10, preferably 1 to 5, and in particular 2 to 4 wt.%, based on the total composition.

11. Composition according to one of claims 1 to 10, characterised in that it contains sodium disulphite in an amount from 0.01 to 0.2, preferably 0.05 to 0.1 wt.%, based on the total composition.

12. Process for producing a composition according to one of claims 1 to 11, characterised in that the active ingredient, the gel former and the other constituents are dissolved in water, optionally with heating and subsequent cooling.

13. Process according to claim 12, characterised in that the solution is carried out under an inert gas atmosphere, in particular under nitrogen.

14. Pharmaceutical composition according to one of claims 1 to 13 for use as an anti-rheumatic, anti-phlogistic and/or anti-inflammatory medicament.

## Claims (Claims for the following Contracting State(s): ES, GR)

1. Process for producing a pharmaceutical composition containing diclofenac sodium in an amount from 0.1 to 10 wt.% for topical application in the form of a gel, which contains as gel former, hydroxypropylcellulose and/or hydroxypropylmethylcellulose in an amount from 1 to 20 wt.%, and propylene glycol in an amount from 15 to 35 wt.%, the composition being free of isopropyl myristate, optionally together with means for adjusting the pH value, stabilisation, preservation and/or conventional pharmaceutical auxiliaries and/or excipients, by dissolving the active ingredient, the gel former and the other constituents in water, optionally with heating and subsequent cooling.

2. Process according to claim 1, characterised in that the composition contains stabilisers, anti-oxidants, solubilisers, reverse-fatting agents, preservatives and/or perfumes.

3. Process according to claim 2, characterised in that the composition contains PEG-7-glyceryl cocoate, isopropyl alcohol and/or sodium disulphite.

4. Process according to claim 3, characterised in that the composition contains the diclofenac sodium in an amount from 0.2 to 5 wt.%, in particular in an amount from 0.5 to 2 wt.%.

5. Process according to one of claims 1 to 4, characterised in that the composition contains diclofenac sodium, hydroxypropylmethylcellulose, propylene glycol, PEG-7-glyceryl cocoate, isopropyl alcohol and sodium disulphite.

6. Process according to one of claims 1 to 5, characterised in that the composition contains a penetration-promoting agent.

7. Process according to one of claims 1 to 6, characterised in that the composition contains ethanol, isopropyl alcohol and/or polyethylene glycol 400 as organic solvent components.

8. Process according to one of claims 1 to 7, characterised in that the composition contains isopropyl alcohol and/or ethanol in an amount from 1 to 35 wt.%, preferably 3 to 20 wt.%, and in particular 3 to 10 wt.%, based on the total composition.

9. Process according to one of claims 1 to 8, characterised in that the composition contains polyethylene glycol 400 in an amount from 1 to 10, preferably 2 to 5 wt.%, based on the total composition.

10. Process according to one of claims 1 to 9, characterised in that the composition contains PEG-7-glyceryl cocoate in an amount from 0.5 to 10, preferably 1 to 5, and in particular 2 to 4 wt.%, based on the total composition.

11. Process according to one of claims 1 to 10, characterised in that the composition contains sodium disulphite in an amount from 0.01 to 0.2, preferably 0.05 to 0.1 wt.%, based on the total composition.

## Revendications (Revendications pour l'(les) Etat(s) contractant(s) suivant(s): AT, BE, CH, DE, DK, FR, GB, IT, LI, LU, NL, SE)

1. Composition pharmaceutique contenant du diclofénac-sodium dans une quantité de 0,1 à 10 % en poids, pour l'utilisation topique sous la forme d'un gel qui contient comme agent de formation du gel de l'hydroxypropylcellulose et/ou de l'hydroxypropylméthylcellulose dans une quantité de 1 à 20 % en poids, ainsi que du propylèneglycol dans une quantité de 15 à 35 % en poids, la composition étant exempte de myristate d'isopropyle, avec le cas échéant des agents pour l'ajustement du pH, la stabilisation, la conservation et/ou d'autres auxiliaires et/ou supports pharmaceutiques habituels.

2. Composition selon la revendication 1, caractérisée en ce qu'elle contient des stabilisants, des antioxydants, des agents solubilisants, des agents de regraissage, des conservateurs et/ou des parfums.

3. Composition selon la revendication 2, caractérisée en ce qu'elle contient un ester PEG-7-glycérylique d'acide gras du coprah, de l'alcool isopropylique et/ou du bisulfite de sodium

4. Composition selon la revendication 3, caractérisée en ce qu'elle cont
dans une quantité de 0,2 à 5 % en poids, en particulier dans une quantité de 0,5 à 2 % en poids.

5. Composition selon l'une des revendications 1 à 4, caractérisée en ce qu'elle contient du diclofénac-sodium, de l'hydroxypropylméthylcellulose, du propylèneglycol, un ester PEG-7-glycérylique d'acide gras du coprah, de l'alcool isopropylique et du bisulfite de sodium.

6. Composition selon l'une des revendications 1 à 5, caractérisée en ce qu'elle contient un agent favorisant la pénétration.

7. Composition selon l'une des revendications 1 à 6, caractérisée en ce qu'elle contient comme solvants organiques constitutifs de l'éthanol, de l'alcool isopropylique et/ou du polyéthylèneglycol 400.

8. Composition selon l'une des revendications 1 à 7, caractérisée en ce qu'elle contient de l'alcool isopropylique et/ou de l'éthanol dans une quantité de 1 à 35 % en poids, de préférence de 3 à 20 % en poids, et en particulier de 3 à 10 % en poids par rapport à la composition totale.

9. Composition selon l'une des revendications 1 à 8, caractérisée en ce qu'elle contient du polyéthylèneglycol 400 dans une quantité de 1 à 10, de préférence de 2 à 5 % en poids, par rapport à la composition totale.

10. Composition selon l'une des revendications 1 à 9, caractérisée en ce qu'elle contient un ester PEG-7-glycérylique d'acide gras du coprah dans une quantité de 0,5 à 10, de préférence de 1 à 5 et en particulier de 2 à 4 % en poids, par rapport à la composition totale.

11. Composition selon l'une des revendications 1 à 10, caractérisée en ce qu'elle contient du bisulfite de sodium dans une quantité de 0,01 à 0,2, de préférence de 0,05 à 0,1 % en poids par rapport à la composition totale.

12. Procédé pour la préparation d'une composition selon l'une des revendications 1 à 11, caractérisé en ce qu'on dissout dans de l'eau la substance active, l'agent de formation du gel et les autres constituants, le cas échéant en chauffant, puis en refroidissant.

13. Procédé selon la revendication 12, caractérisé en ce qu'on effectue la dissolution sous une atmosphère de gaz inerte, en particulier sous azote.

14. Composition pharmaceutique selon l'une des revendications 1 à 13 pour l'utilisation comme médicament antirhumatismal, antiphlogistique et/ou antiinflammatoire.

## Revendications (Revendications pour l'(les) Etat(s) contractant(s) suivant(s): ES, GR)

1. Procédé de préparation d'une composition pharmaceutique contenant du diclofénac-sodium dans une quantité de 0,1 à 10 % en poids, pour l'utilisation topique sous la forme d'un gel, qui contient comme agent de formation du gel de l'hydroxypropylcellulose et/ou de l'hydroxypropylméthylcellulose dans une quantité de 1 à 20 % en poids, ainsi que du propylèneglycol dans une quantité de 15 a 35 % en poids, la composition étant exempte de myristate d'isopropyle, le cas échéant avec des agents pour l'ajustement du pH, la stabilisation, la conservation et/ou des substances auxiliaires et/ou des supports pharmaceutiques habituels, en dissolvant dans de l'eau la substance active, l'agent de formation du gel et les autres constituants, le cas échéant en chauffant puis en refroidissant.

2. Procédé selon la revendication 1, *caractérisé* en ce que la composition contient des stabilisants, des antioxydants, des agents de solubilisation, des agents de regraissage, des conservateurs et/ou des parfums.

3. Procédé selon la revendication 2, *caractérisé* en ce que la composition contient un ester PEG-7-glycérylique d'acide gras du coprah, de l'alcool isopropylique et/ou du bisulfite de sodium.

4. Procédé selon la revendication 3, *caractérisé* en ce que la composition contient le diclofénac-sodim dans une quantité de 0,2 à 5 % en poids, en particulier dans une quantité de 0,5 à 2 % en poids.

5. Procédé selon l'une des revendications 1 à 4, *caractérisé* en ce que la composition contient du diclofénac-sodium, de hydroxypropylméthylcellulose, du propylèneglycol, des esters d'acide gras du coprah et du PEG-7-glycérol, de l'alcool isopropylique et du bisulfite de sodium.

6. Procédé selon l'une des revendications 1 à 5, *caractérisé* en ce que la composition contient un agent favorisant la pénétration.

7. Procédé selon l'une des revendications 1 à 6, *caractérisé* en ce que la composition contient comme solvants organiques constitutifs de l'éthanol, de l'alcool isopropylique et/ou du polyéthylèneglycol 400.

8. Procédé selon l'une des revendications 1 à 7, *caractérisé* en ce que la composition contient de l'alcool isopropylique et/ou de l'éthanol dans une quantité de 1 à 35 % en poids, de préférence de 3 à 20 % en poids, et en particulier de 3 à 10 % en poids, par rapport à la composition totale.

9. Procédé selon l'une des revendications 1 à 8, *caractérisé* en ce que la composition contient du polyéthylèneglycol 400 dans une quantité de 1 à 10, de préférence de 2 à 5 % en poids, par rapport à la composition totale.

10. Procédé selon l'une des revendications 1 à 9, *caractérisé* en ce que la composition contient un ester PEG-7-glycérylique d'acide gras du coprah dans un quantité de 0,5 à 10, de préférence de 1 à 5 % et en particulier de 2 à 4 % en poids, par rapport à la composition totale.

11. Procédé selon l'une des revendications 1 à 10, *caractérisé* en ce que la composition contient du bisulfite de sodium dans une quantité de 0,01 à 0,2, de préférence de 0,05 à 0,1 % en poids par rapport à la composition totale.
